(19) Europäisches Patentamt
European Patent Office
Office européen des brevets

(11) EP 0 605 503 B1

(12) **EUROPÄISCHE PATENTSCHRIFT**

(45) Veröffentlichungstag und Bekanntmachung des Hinweises auf die Patenterteilung:
**30.10.1996 Patentblatt 1996/44**

(21) Anmeldenummer: **92919591.5**

(22) Anmeldetag: **16.09.1992**

(51) Int. Cl.⁶: **A61K 7/06**

(86) Internationale Anmeldenummer:
**PCT/EP92/02118**

(87) Internationale Veröffentlichungsnummer:
**WO 93/05756 (01.04.1993 Gazette 1993/09)**

# (54) ZUBEREITUNGEN ZUR HAARBEHANDLUNG

HAIR-TREATMENT PREPARATIONS

PREPARATIONS POUR LE TRAITEMENT DES CHEVEUX

(84) Benannte Vertragsstaaten:
**AT BE CH DE DK ES FR GB GR IE IT LI NL PT SE**

(30) Priorität: **25.09.1991 DE 4131898**

(43) Veröffentlichungstag der Anmeldung:
**13.07.1994 Patentblatt 1994/28**

(73) Patentinhaber: **Henkel Kommanditgesellschaft auf Aktien**
**40191 Düsseldorf (DE)**

(72) Erfinder:
- **MÜLLER, Reinhard**
  **D-5140 Erkelenz 7 (DE)**
- **SEIDEL, Kurt**
  **D-4000 Düsseldorf 13 (DE)**
- **HOLLENBERG, Detlef**
  **D-4006 Erkrath (DE)**

(56) Entgegenhaltungen:
**EP-A- 0 356 665**
**WO-A-91/03229**
**US-A- 4 374 125**

Bemerkungen:
Verbunden mit 92115820.0/0535447 (europäische Anmeldenummer/Veröffentlichungsnummer) durch Entscheidung vom 11.04.95.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.13.8/3.4

**Beschreibung**

Die Erfindung betrifft Zubereitungen zur Haarbehandlung, die eine Kombination aus einem zwitterionischen Polymerisat und einer Pyrrolidoncarbonsäure enthalten.

Jede reinigende oder kosmetische Behandlung der Haare stellt einen Eingriff in die natürliche Struktur des Haares dar. Dies hat zur Folge, daß die Haare nach einer solchen Behandlung, wie sie beispielsweise das Waschen, Färben oder Dauerwellen darstellt, häufig neben den gewünschten Änderungen auch eine Reihe von unbefriedigenden Eigenschaften aufweisen. Neben einer verschlechterten Naß- und Trockenkämmbarkeit seien hier die elektrostatische Aufladung und das mangelhafte Feuchthaltevermögen genannt; häufig ist auch das Lockenhaltevermögen der trockenen Haare unzureichend. Weiterhin ist es gewünscht, daß die Kopfhaut durch die Haarbehandlung möglichst wenig in Mitleidenschaft gezogen wird.

Um diesem Mißstand abzuhelfen, müssen entweder den Haarbehandlungsmitteln entsprechende Komponenten hinzugefügt oder die Haare anschließend einer separaten Behandlung mit diesen Substanzen, die dann üblicherweise in Form einer Spülung formuliert werden, unterzogen werden.

Als solche haareigenschaftsverbessernde Wirkstoffe werden häufig polymere Verbindungen, insbesondere kationische, anionische und zwitterionische Polymerisate, eingesetzt. Entsprechende Polymerisate sind beispielsweise in den Patentanmeldungen bzw. Patenten US 3,816,616, US 3,472,840, DE-A-21 50 557, DE-A-28 17 369, DE-A-28 11 010, DE-A-33 01 121, DE-A-30 44 738, DE-A-37 08 451, GB-B-2 104 091 und DE-A-39 29 973 offenbart.

2-Pyrrolidon-5-carbonsäure ist Bestandteil des sogenannten natürlichen Feuchthaltefaktors ("Natural Moisturizing Factor", NMF) der Haut. Diese Verbindung bzw. ihr Natriumsalz wird daher als Feuchthaltemittel für Hautcremes und andere kosmetische Produkte eingesetzt (vgl. z.B. US-A-4 374 125). Weiterhin ist bekannt, daß die Salze die Weichheit und Elastizität der Hornschicht sowie der Haaroberfläche erhöhen (H.P.Fiedler, Lexikon der Hilfsstoffe für Pharmazie, Kosmetik und angrenzende Gebiete, 3. Auflage, Editio Cantor Aulendorf, (1989), S. 110, 111).

Es wurde nun gefunden, daß die Wirkungen beider Stoffklassen in Haarbehandlungsmitteln in synergistischer Weise gesteigert werden, wenn diese Verbindungen in Kombination eingesetzt werden.

Gegenstand der Erfindung sind daher Zubereitungen, enthaltend übliche Bestandteile zur Behandlung der Haare, dadurch gekennzeichnet, daß sie eine Kombination aus

A) einem zwitterionischen Polymerisat und
B) einer Verbindung der allgemeinen Formel (I),

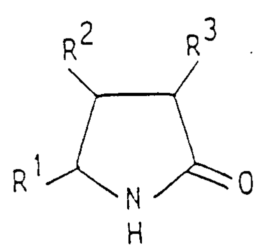

(I)

in der mindestens einer der Substituenten $R^1$ bis $R^3$ steht für eine Gruppe $-COOR^4$, in der $R^4$ Wasserstoff, ein Alkalimetallion, ein Erdalkalimetallion oder ein Ammoniumion $^+NHR^5R^6R^7$, in dem $R^5$ bis $R^7$ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist, und die restlichen Substituenten $R^1$ bis $R^3$ für Wasserstoff oder Alkylgruppen mit 1 bis 4 C-Atomen stehen, enthalten.

Unter zwitterionischen Polymerisaten im Sinne der Erfindung werden solche Polymerisate verstanden, die sowohl kationische als auch anionische Gruppen oder leicht in diese überführbare Gruppen enthalten.

Kationische Gruppen sind beispielsweise Gruppen, die mindestens eine Ammonium- oder Phosphonium-Einheit enthalten; Beispiele für eine leicht in eine kationische Gruppe überführbare Einheit sind primäre, sekundäre und insbesondere tertiäre Amino-Gruppen. Ammoniumgruppen sind bevorzugte kationische Gruppen.

Als anionische Gruppen kommen beispielsweise Carbon-, Sulfon- und Phosphorsäuregruppen in freier Form oder in Salzform in Betracht. Die Carbonsäuregruppen und ihre Alkali-, Erdalkali-, Aluminium- und Ammoniumsalze sind bevorzugte anionische Gruppen.

Die erfindungsgemäß eingesetzten Polymerisate können aus Monomeren aufgebaut sein, die sowohl kationische als auch anionische Gruppen enthalten. Die Polymerisate können in diesem Fall aus einem einzigen Monomertyp bestehen. Es ist aber auch möglich, Copolymere aus den genannten Monomeren und weiteren nichtionischen Monomeren einzusetzen. Solche nichtionischen Monomere können beispielsweise Ester und Amide auf Vinylbasis, wie

Methylacrylat, Ethylacrylat, Methylmethacrylat, Ethylmethacrylat, Hydroxypropylmethacrylat und Acrylamid sein. Bevorzugt enthalten diese Copolymerisate mindestens 20 %, insbesondere mindestens 50 %, an ionischen Monomeren.

Im Rahmen der Erfindung bevorzugte Polymerisate sind aus mindestens 2 ionischen Monomeren, einem kationischen und einem anionischen Monomer, aufgebaut. Kationische und anionische Monomere können dabei in einem Molverhältnis 1:1 vorliegen. Es ist jedoch bevorzugt, einen Monomertyp, insbesondere die kationischen Monomeren, im Überschuß einzusetzen. In der Regel werden kationische und anionische Monomere im Verhältnis 95:5 bis 60:40 eingesetzt. Weiterhin können die Polymeren nichtionische Monomere der oben genannten Art in Mengen bis zu 50 Mol-%, insbesondere bis zu 20 Mol-% enthalten.

Erfindungsgemäß bevorzugt sind die in der DE-A-39 29 973 beschriebenen zwitterionischen Polymerisate, die sich im wesentlichen zusammensetzen aus

α) Monomeren mit quartären Ammoniumgruppen der allgemeinen Formel (II),

$$R^8\text{-}CH{=}CR^9\text{-}CO\text{-}Z\text{-}(C_nH_{2n})\text{-}N^{(+)}R^{10}R^{11}R^{12}\ A^{(-)} \qquad \text{(II)}$$

in der $R^8$ und $R^9$ unabhängig voneinander stehen für Wasserstoff oder eine Methylgruppe und $R^{10}$, $R^{11}$ und $R^{12}$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist

und

β) monomeren Carbonsäuren der allgemeinen Formel (III),

$$R^{13}\text{-}CH{=}CR^{14}\text{-}COOH \qquad \text{(III)}$$

in denen $R^{13}$ und $R^{14}$ unabhängig voneinander Wasserstoff oder Methylgruppen sind, oder Alkali-, Erdalkali-, Aluminium- oder Ammoniumsalzen dieser Säuren.

Geeignete Ausgangsmonomere für die Monomere (α) sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die Herstellung der genannten Monomeren erfolgt nach bekannten Verfahren, wie sie z. B. in US 3,878,247, DE-C-28 19 735, DE-C-28 36 520, DE-C-34 02 599 oder CH 464 891 beschrieben sind.

Die eine tertiäre Aminogruppe enthaltenden Monomeren werden dann in bekannter Weise quaterniert, wobei als Alkylierungsreagenzien Methylchlorid, Dimethylsulfat oder Diethylsulfat besonders geeignet sind. Die Quaternisierungsreaktion kann in wäßriger Lösung oder im Lösungsmittel erfolgen. Geeignete Verfahren sind z. B. in DE-A-33 30 326, DE-A-25 37 378 und DE-A-32 44 274 beschrieben.

Vorteilhafterweise werden solche Monomere der Formel (II) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (II), bei denen $R^{10}$, $R^{11}$ und $R^{12}$ Methylgruppen sind.

Das Acrylamidopropyl-trimethylammoniumchlorid ist ein ganz besonders bevorzugtes Monomer der Formel (II).

Monomere Carbonsäuren (β) sind Acrylsäure, Methacrylsäure, Crotonsäure und 2-Methyl-crotonsäure. Bevorzugt werden Acryl- oder Methacrylsäure, insbesondere Acrylsäure, eingesetzt. Bevorzugte eingesetzte Salze dieser Carbonsäuren sind das Lithium-, Natrium-, Kalium-, Magnesium-, Calcium- und Aluminiumsalz. Besonders bevorzugt ist das Natriumsalz. Weiterhin können die Ammoniumsalze eingesetzt werden, bei denen das Ammoniumion ein bis drei Alkylgruppen mit 1-4 Kohlenstoffatomen oder Hydroxyalkylgruppen mit 2-4 Kohlenstoffatomen als Substituenten haben kann. Salze mit unsubstituierten Ammoniumionen und Triethanolammoniumionen sind bevorzugt.

Bezüglich der Herstellung dieser zwitterionischen Polymere wird ausdrücklich auf die Offenbarung der DE-A-39 29 973 Bezug genommen.

Die Verbindungen der allgemeinen Formel (I) sind Derivate des 2-Pyrrolidons.

Bevorzugte Derivate sind die 2-Pyrrolidon-3-, -4- und -5-carbonsäure und deren Salze. Bevorzugte Salze dieser Verbindungen sind die Natrium-, Kalium-, Calcium-, Magnesium- und solche Ammoniumsalze, bei denen das Ammoniumion neben Wasserstoff eine bis drei $C_1$- bis $C_4$-Alkylgruppen trägt.

Besonders bevorzugte Verbindungen der allgemeinen Formel (I) sind die 2-Pyrrolidon-5-carbonsäure und deren Salze. Das Natriumsalz ist ganz besonders bevorzugt.

Die zwitterionischen Polymerisate sind in den erfindungsgemäßen Zubereitungen vorzugsweise in Mengen von 0,1 bis 10 Gew.-%, bezogen auf die gesamte Zubereitung, enthalten. Konzentrationen von 0,2 bis 5 Gew.-% sind besonders bevorzugt.

Die Verbindungen der Formel (I) sind in den erfindungsgemäßen Zubereitungen bevorzugt in Mengen von 0,1 bis 15 Gew.-%, insbesondere von 0,5 bis 10 Gew.-%, bezogen auf die gesamte Zubereitung, enthalten.

Neben den zwitterionischen Polymerisaten und den Verbindungen der allgemeinen Formel (I) können die erfindungsgemäßen Zubereitungen alle in Haarbehandlungsmitteln üblichen Bestandteile enthalten. Dabei hängt es von der Art des Mittels ab, welche der im folgenden genannten Bestandteile im Mittel enthalten sind.

Die meisten Haarbehandlungsmittel, wie Shampoos, Spülungen, Dauerwellmittel, Haarfärbemittel und Haartönungsmittel sind wäßrige Zubereitungen mit einem Gehalt an oberflächenaktiven Verbindungen. Als oberflächenaktive Verbindungen werden dabei anionische, kationische, zwitterionische, amphotere und/oder nichtionischen Tenside eingesetzt.

Als anionische Tenside eignen sich in erfindungsgemäßen Haarbehandlungsmitteln alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind die Natrium-, Kalium- und Ammonium- sowie die Mono-, Di- und Trialkanolammoniumsalze mit 2 oder 3 C-Atomen in der Alkanolgruppe, von

- linearen Fettsäuren mit 10 bis 22 C-Atomen (Seifen),
- Ethercarbonsäuren der Formel $R\text{-}O\text{-}(CH_2\text{-}CH_2O)_x\text{-}CH_2\text{-}COOH$, in der R eine lineare Alkylgruppe mit 10 bis 22 C-Atomen und x = 0 oder 1 bis 10 ist,
- Acylsarcosiden mit 10 bis 18 C-Atomen in der Acylgruppe,
- Acyltauriden mit 10 bis 18 C-Atomen in der Acylgruppe,
- Acylisethionaten mit 10 bis 18 C-Atomen in der Acylgruppe,
- Sulfobernsteinsäuremono- und dialkylestern mit 8 bis 18 C-Atomen in der Alkylgruppe und Sulfobernsteinsäuremono-alkylpolyoxyethylestern mit 8 bis 18 C-Atomen in der Alkylgruppe und 1 bis 6 Oxyethylgruppen,
- linearen Alkansulfonaten mit 12 bis 18 C-Atomen,
- linearen Alpha-Olefinsulfonaten mit 12 bis 18 C-Atomen,
- Alpha-Sulfofettsäuremethylestern von Fettsäuren mit 12 bis 18 C-Atomen,
- Alkylsulfaten und Alkylpolyglykolethersulfaten der Formel $R\text{-}O(CH_2\text{-}CH_2O)_x\text{-}OSO_3H$, in der R eine bevorzugt lineare Alkylgruppe mit 10 bis 18 C-Atomen und x = 0 oder 1 bis 12 ist,
- Gemischen oberflächenaktiver Hydroxysulfonate gemäß DE-A-37 25 030
- sulfatierten Hydroxyalkylpolyethylen- und/oder Hydroxyalkylenpropylenglykolethern gemäß DE-A-37 23 354.
- Sulfonate ungesättigter Fettsäuren mit 12 bis 24 C-Atomen und 1 bis 6 Doppelbindungen gemäß DE-A-39 26 344,
- Estern der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate und Alkylpolyglykolethersulfate mit 10 bis 18 C-Atomen in der Alkylgruppe und bis zu 12 Glykolethergruppen im Molekül.

Shampoos auf Basis anionischer Tenside sind bevorzugte erfindungsgemäße Haarbehandlungsmittel.

Als kationische Tenside sind in den erfindungsgemäßen Haarbehandlungsmitteln bevorzugt quartäre Ammoniumverbindungen wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid enthalten. Beispiele für weitere geeignete kationische Tenside sind Cetylpyridiniumchlorid, Talgalkyltris-(oligooxyalkyl)-ammonium-phosphat sowie die in DE-A-34 42 175 offenbarten Verbindungen..

Haarspülungen auf Basis kationischer Tenside sind ebenfalls bevorzugte erfindungsgemäße Haarbehandlungsmittel.

Als zwitterionische Tenside werden solche oberflächenaktiven Verbindungen bezeichnet, die im Molekül mindestens eine quartäre Ammoniumgruppe und mindestens eine $-COO^{(-)}$- oder $-SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das $C_{12-18}$-Acylsarcosin.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise

- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- $C_{12}$-$C_{22}$-Fettsäuremono- und -diester von Anlagerungsprodukten von 1 bis 30 Mol Ethylenoxid an Glycerin,
- Glycerinmono- und -diester und Sorbitanmono- und -diester von gesättigten und ungesättigten $C_8$-$C_{22}$-Fettsäuren und deren Ethylenoxidanlagerungsprodukte,
- $C_8$-$C_{22}$-Alkylmono- und -oligoglycoside und deren ethoxylierte Analoga und
- Anlagerungsprodukte von 5 bis 60 Mol Ethylenoxid an Ricinusöl und gehärtetes Ricinusöl.

Besonders bevorzugte nichtionogene Tenside sind $C_8$-$C_{22}$-Alkylmono- und -oligoglycoside, deren Herstellung und ihre Verwendung als oberflächenaktive Stoffe sind beispielsweise aus US 3,839,318, US 3,707,535, US 3,547,828, DE-A-19 43 689, DE-A-20 36 472 und DE-A-30 01 064 sowie EP-A-77 167 bekannt ist. Die Herstellung erfolgt insbesondere durch Umsetzung von Glucose oder Oligosacchariden mit primären Alkoholen mit 8 bis 22 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Oligomerisierungsgrade von 1,4 und kleiner sind besonders bevorzugt. Der Oligomerisierungsgrad ist dabei ein statistischer Mittelwert, dem eine für solche technischen Produkte übliche Homologenverteilung zugrunde liegt.

Es wurde gefunden, daß die Wirkung der erfindungsgemäßen Wirkstoffkombination noch gesteigert wird, wenn das Haarbehandlungsmittel zusätzlich ein nichtionogenes Tensid enthält. Insbesondere ist dies der Fall, wenn das nichtionogene Tensid ein $C_8$-$C_{22}$-Alkylmono- und -oligoglycosid ist.

Im Rahmen der vorliegenden Erfindung sind daher Zubereitungen besonders bevorzugt, die zusätzlich ein nichtionogenes Tensid, insbesondere ein $C_8$-$C_{22}$-Alkylmono- und -oligoglucosid, enthalten.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen und tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Vorzugsweise enthalten die erfindungsgemäßen Zubereitungen oberflächenaktive Verbindungen in Mengen von 0,1 bis 50 Gew.-%, bezogen auf die gesamte Zubereitung.

Weitere übliche Bestandteile von Haarbehandlungsmitteln sind:

- kationische, anionische und nichtionische Polymere, wie beispielsweise quaternisierte Celluloseether, Polysiloxane, Vinylpyrrolidon-Copolymere, Acrylamid-Dimethyldiallylammoniumchlorid-Copolymere, VinylacetatCrotonsäureCopolymere, Polyvinylpyrrolidon und Polyacrylsäuren,
- Verdickungsmittel, wie beispielsweise Celluloseether, unvernetzte und mit Polyolen vernetzte Polyacrylsäuren, Agar-Agar, Guar-Gum, Alginate und Xanthan-Gum,
- Proteinhydrolysate, insbesondere Elastin- und Kollagen-Hydrolysate,
- Parfümöle,
- Lösungsvermittler, wie Ethanol, Isopropanol, Ethylenglykol, Propylenglykol, Glycerin und Diethylenglykol,
- Farbstoffe,
- direktziehende Farbstoffe zum Färben und Tönen der Haare,
- Vorläufer für Oxidationsfarbstoffe (Entwickler- und Kupplerkomponenten) zum Färben und Tönen der Haare,

- Reduktionsmittel wie Thioglykolsäure zum Spalten der Disulfid-Brücken bei der Dauerwelle,
- Oxidationsmittel wie Kaliumbromat und Wasserstoffperoxid zur Fixierung im Rahmen der Dauerwelle,
- Antischuppenwirkstoffe wie Piroctone Olamine und Zink Omadine,
- Substanzen zur Einstellung des pH-Wertes wie Citronensäure/Natriumcitrat-Puffer,
- Wirkstoffe wie Panthenol, Allantoin, Pflanzenextrakte und Vitamine
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Überfettungsmittel wie polyethoxylierte Lanolinderivate, Lecithinderivate und Fettsäurealkanolamide
- Komplexbildner wie EDTA und Phosphonsäuren,
- Quell- und Penetrationsstoffe wie Glycerin, Propylenglykolmonoethylether, Guanidine und Harnstoff,
- Trübungsmittel wie Latex,
- Perlglanzmittel wie Ethylenglykolmono- und -distearat,
- Treibmittel wie Propan-Butan-Gemische, $N_2O$, Dimethylether und Luft, sowie
- Antioxidantien.

Die erfindungsgemäßen Zubereitungen weisen bevorzugt einen pH-Wert von 3,5 bis 10, insbesondere von 4,5 bis 9, auf.

Haarbehandlungsmittel, in denen die erfindungsgemäßen Zubereitungen verwendet werden können, sind beispielsweise Haarshampoos, Duschbäder, Haarnachspülmittel, Haarfärbemittel, Haartönungsmittel, Dauerwellmittel, Dauerwell-Fixiermittel, Haarfestiger, Haar-Fönwellmittel, Haarkuren, Haarcremes, Haarlotionen und Haarwässer.

Diese Mittel können in allen für Haarbehandlungsmittel üblichen Konfektionierungen, wie beispielsweise in Form einer wäßrigen Lösung oder Emulsion, einer wäßrig-alkoholischen oder alkoholischen Lösungen, einer Creme, eines Gels, einer Lotion oder eines Aerosols vorliegen.

Ebenfalls Gegenstand der Erfindung ist die Verwendung von Zubereitungen nach einem der Ansprüche 1 bis 11 zur Behandlung, insbesondere zur Reinigung und Pflege, von Haaren. Dabei werden sowohl Behandlungen umfaßt, bei denen das Haar nach der Behandlung gespült wird, als auch solche, bei denen das Mittel auf dem Haar verbleibt. Als besonders vorteilhaft haben sich die erfindungsgemäßen Zubereitungen in solchen Fällen erwiesen, bei denen das Haar nach einer bestimmten Einwirkzeit der Haarbehandlungsmittels gespült wird.

## Beispiele

Für die Untersuchungen wurden Shampoos folgender Zusammensetzung (in Gew.-%) verwendet:

| | V | S1 | S2 | S3 | S4 | S5 | S6 | S7 | S8 | S9 |
|---|---|---|---|---|---|---|---|---|---|---|
| Texapon[R]N 25[1] | 9,0 | 9,0 | 9,0 | 9,0 | 9,0 | 9,0 | 9,0 | 9,0 | 9,0 | 9,0 |
| Akypo[R] RLM 100 NV[2] | 3,0 | 3,0 | 3,0 | 3,0 | 3,0 | 3,0 | 3,0 | 3,0 | 3,0 | 3,0 |
| Dehyton[R]K[3] | 3,0 | 3,0 | 3,0 | 3,0 | 3,0 | 3,0 | 3,0 | 3,0 | 3,0 | 3,0 |
| Nutrilan[R] I[4] | 0,5 | 0,5 | 0,5 | 0,5 | 0,5 | 0,5 | 0,5 | 0,5 | 0,5 | 0,5 |
| Polymerisat P1[5] | - | 0,5 | 1,0 | - | - | 0,5 | 0,5 | 0,5 | 1,0 | 1,0 |
| Ajidew[R]N50[6] | - | - | - | 5,0 | 10,0 | 5,0 | 10,0 | 5,0 | 5,0 | 5,0 |
| Alkylglucosid APG-600[7] | - | - | - | - | - | - | - | 2,0 | - | 2,0 |
| Wasser ad | 100 | 100 | 100 | 100 | 100 | 100 | 100 | 100 | 100 | 100 |

[1] Natriumlaurylethersulfat; CTFA-Bezeichnung: Sodium Laureth Sulfate (ca. 28 % Aktivsubstanz in Wasser) (HENKEL)
[2] $C_{12-14}$-Alkyl-O-$(CH_2$-$CH_2$-$O)_{10}$-$CH_2$-COONa (22 % Aktivsubstanz in Wasser) (CHEM-Y)
[3] Fettsäureamid-Derivat mit Betainstruktur der Formel R-CONH-$(CH_2)_3$-$N^+(CH_3)_2$-$CH_2$-$COO^-$; CTFA-Bezeichnung: Cocamidopropyl Betaine (ca. 30 % Aktivsubstanz in Wasser) (HENKEL)
[4] Eiweiß-Hydrolysat; CTFA-Bezeichnung Hydrolyzed-Animal-Collagen (GRÜNAU)
[5] Mit Natronlauge neutral gestelltes Polymer aus Acrylamidopropyltrimethylammoniumchlorid und Acrylsäure im Molverhältnis 3:1, gemäß DE-A-39 29 973. Hinsichtlich der Herstellung des Polymerisats wird ausdrücklich auf die genannte Druckschrift Bezug genommen.
[6] Natriumsalz der dl-2-Pyrrolidon-5-carbonsäure (50%-ige wäßrige Lösung) (AJINOMOTO).
[7] Wäßrige Lösung von $RO(Z)_x$ mit Z=Glucose, x=1,4 und R= n-Alkyl($C_{12-14}$), (50 % Aktivsubstanz) (HORIZON)

Es wurden das Feuchthaltevermögen, das Lockenhaltevermögen ("Curl retention"-Wert) sowie die Quellung von Schweineepidermis, die ein Maß für die Hautfreundlichkeit der Zubereitung darstellt, bestimmt.

### 1. Feuchthaltevermögen

Das Feuchthaltevermögen ist ein Maß dafür, in welchem Umfang der natürliche Wassergehalt des Haares in trokkener Umgebung erhalten bleibt.

Die Bestimmung erfolgte an standardisiertem mittelbraunem europäischem Haar (Alkinco 6634; Strähnengewicht: 2 g).

Vorbehandlung:

Zunächst wurden die Haarsträhnen mit einer wäßrigen Lösung von Texapon N25 (12 % Aktivsubstanz) gewaschen und luftgetrocknet.

Bestimmung des Nullwertes:

Die Haarsträhnen wurden 24 Stunden bei Raumtemperatur und 95 % rel. Luftfeuchtigkeit belassen. Das anschließend bestimmte Gewicht der Haare wurde mit $m_H$ bezeichnet. Anschließend wurden die Haarsträhnen bei Raumtemperatur 24 Stunden über Phosphorpentoxid getrocknet. Die dabei von den Haaren abgegebene Wassermenge, die aus der Gewichtszunahme des Phosphorpentoxids bestimmt wurde, wurde mit $m_W$ bezeichnet. Schließlich wurden die Haarsträhnen bei 50°C im Umlufttrockenschrank ca. 30 Minuten bis zur Gewichtskonstanz nachgetrocknet; das Gewicht der trockenen Haare betrug $m_{Ho}$.

Messung:

Dieselben Haarsträhnen wurden 5 Minuten mit dem Shampoo behandelt und anschließend 30 Sekunden mit Wasser abgespült. Dieser Vorgang wurde fünfmal wiederholt. Danach wurden wie bei der Bestimmung des Nullwertes die Größen $m_H$, $m_W$ und $m_{Ho}$ bestimmt.

Für jede Bestimmung wurden 15 Haarsträhnen untersucht.

Als Ergebnisse der Messungen sind in Tabelle 1 dargestellt:
Wasseraufnahme ($m_H$-$m_{Ho}$)
Restwassergehalt ($m_H$-$m_{Ho}$)-$m_W$

Die bei der Nullmessung (Wassergehalt bei 95 % rel. Luftfeuchtigkeit: 18 % des Haargewichtes; Wassergehalt nach der Lagerung über Phosphorpentoxid: 3 % des Haargewichtes) erhaltenen Werte wurden gleich 100 % gesetzt.

Tabelle 1

| Feuchthaltevermögen | | |
|---|---|---|
| Shampoo | Wasseraufnahme | Restwassergehalt |
| Nullwert | 100 % | 100 % |
| V | 100 % | 100 % |
| S1 | 100 % | 100 % |
| S3 | 109 % | 113 % |
| S4 | 108 % | 127 % |
| S5 | 110 % | 121 % |
| S6 | 112 % | 135 % |

**2. Hautverträglichkeits-Untersuchungen**

Zur Bestimmung der Hautverträglichkeit der Shampoos wurde die von Zeidler und Reese entwickelte in-vitro-Methode verwendet, die in der Zeitschrift Ärztliche Kosmetologie 13, 39-45 (1983) ausführlich dargestellt ist.

Als Maß für die Hautverträglichkeit der Shampoos diente die Quellung von Schweine-Epidermis. Dazu wurde die benötigte Epidermis unmittelbar nach der Schlachtung junger Schweine gewonnen und tiefgekühlt gelagert.

Für die Messung wurden ausgestanzte Epidermisstreifen der Größe 1 cm x 6 cm 30 Minuten lang in die Shampoos (jeweils auf einen Tensidgehalt von 2 Gew.-% mit Wasser verdünnt) getaucht, die auf 39°C temperiert und auf pH = 6,5 eingestellt waren. Sodann wurde nach kurzem Spülen und Entfernen des anhaftenden Wasser durch leichtes Abpressen unter definierten Bedingungen das Gewicht der gequollenen Streifen bestimmt. Anschließend wurden die Streifen 24 Stunden über Calciumchlorid entwässert und erneut gewogen. Um Einflüsse auszuschalten, die auf spezifische Eigenschaften des jeweiligen Tieres oder den Entnahmeort (Rücken, Seite) zurückgehen, wurde jeweils eine Standardmessung durchgeführt. Dabei wurde ein unmittelbar benachbarter Epidermisstreifen in gleicher Weise mit Wasser anstelle des Shampoos behandelt.
Die Meßwerte s für die Shampoos-Behandlung und w für die Behandlung mit Wasser ergeben sich aus der Beziehung:

$$s, w = \frac{\text{Gewicht(gequollene Epidermis) - Gewicht(trockene Epidermis)}}{\text{Gewicht(trockene Epidermis)}}$$

Die standardisierte, relative Quellungsänderung Q ist schließlich definiert als

$$Q = \left(\frac{s}{w} - 1\right) * 100\ \%$$

Der Q-Wert der wasserbehandelten Haut ist somit definitionsgemäß 0 %; negative Werte weisen auf quellungshemmende Eigenschaften hin. Die Ergebnisse der Quellungsmessungen sind in Tabelle 2 zusammengestellt.

Tabelle 2

| Quellungsänderungen | |
|---|---|
| Shampoo | Q-Wert |
| V | 50 ± 7 |
| S1 | 46 ± 6 |
| S3 | 40 ± 5 |
| S5 | 33 ± 6 |
| S7 | 24 ± 7 |

**3. Lockenhaltevermögen**

Eine 15 cm lange Haarsträhne (Alkinco 6634; Strähnengewicht: 2 g) wurde auf ein Glasrohr mit einem Außendurchmesser von 1,7 cm gewickelt, fixiert und mit 0,2 g des Shampoos behandelt. Anschließend wurde die Haarsträhne mit Wasser gespült und getrocknet. Ein Maß für die Stabilität der nach dem Herausziehen des Glasstabes erhaltenen Locke ist der Curl-Retention-Wert. Der Curl-Retention-Wert ist definiert als $[(l-l_x)/(l-l_o)] * 100\ \%$ , wobei l die Länge der Haarsträhne (15 cm), $l_o$ die Länge der Haarlocke unmittelbar nach dem Trocknen und $l_x$ die Länge der Haarlocke nach 48 h Lagerung in einem Trockenschrank bei konstanten Bedingungen (30 °C, 40 % relative Luftfeuchtigkeit) ist. Die Ergebnisse der Messungen sind in Tabelle 3 zusammengestellt.

Tabelle 3

| Curl-Retention-Werte | |
|---|---|
| Shampoo | Curl-Retention-Wert |
| V | 87,1 |
| S2 | 94,7 |
| S3 | 85 |
| S8 | 96,2 |
| S9 | 96,8 |

## 4. Anwendungsbeispiele

### 4.1. Haarspülung

| Komponenten | Gew.-% |
|---|---|
| Texapon[R] N 25 | 5,0 |
| Dehyton[R] K | 1,0 |
| Polymerisat P1[8] | 2,5 |
| Ajidew[R] N50 | 2,0 |
| Wasser | ad 100 |

[8] 20 % Aktivsubstanz in Wasser

### 4.2. Haarspülung

| Komponenten | Gew.-% |
|---|---|
| Texapon[R] N 25 | 5,0 |
| Dehyton[R] AB 30[9] | 1,0 |
| Polymerisat P1[8] | 3,0 |
| Ajidew[R] N50 | 3,0 |
| Wasser | ad 100 |

[8] 20 % Aktivsubstanz in Wasser

[9] Fettamin-Derivat mit Betainstruktur; CTFA-Bezeichnung: Coco-Betaine (ca. 30 % Aktivsubstanz in Wasser) (HENKEL)

4.3. Haarspülung

| Komponenten | Gew.-% |
|---|---|
| Texapon[R]SB 3[10] | 0,9 |
| Texapon[R]K14 S spez.[11] | 1,2 |
| Dehyton[R]AB 30 | 1,0 |
| Polymerisat P1[8] | 1,5 |
| Ajidew[R]N50 | 2,0 |
| Ajidew[R]N50 | 2,0 |
| Wasser | ad 100 |

[8] 20 % Aktivsubstanz in Wasser
10 Sulfobernsteinsäurehalbester auf Basis eines $C_{12-14}$-Alkylpoly(3 EO)glykolethers, Dinatriumsalz; CTFA-Bezeichnung: Disodiumlaurethsulfosuccinate (40 % Aktivsubstanz in Wasser) (HENKEL)
[11] Natriumlaurylmyristylethersulfat (ca. 30 % Aktivsubstanz in Wasser (HENKEL)

4.4. Haarspülung

| Komponenten | Gew.-% |
|---|---|
| Texapon[R]SB 3 | 1,0 |
| Dehyton[R]AB 30 | 7,5 |
| Polymerisat P1[8] | 2,5 |
| Ajidew[R]N50 | 2,0 |
| Wasser | ad 100 |

[8] 20 % Aktivsubstanz in Wasser

4.5. Haarspülung

| Komponenten | Gew.-% |
|---|---|
| $C_{16-18}$-Fettalkohol | 3,0 |
| Dehyton$^R$K | 8,0 |
| Texapon$^R$N 25 | 5,0 |
| Polymerisat P1[8] | 2,5 |
| Ajidew$^R$N50 | 5,0 |
| Wasser | ad 100 |

[8] 20 % Aktivsubstanz in Wasser

Zur Herstellung dieser Haarspülung wurde die Mischung aus Tensiden und Polymerisat in die geschmolzene Fettphase gegeben und emulgiert.

4.6. Haarshampoo

| Komponenten | Gew.-% |
|---|---|
| Texapon$^R$N 25 | 50,0 |
| Dehyton$^R$K | 10,0 |
| Polymerisat P1[8] | 5,0 |
| Ajidew$^R$N50 | 10,0 |
| Wasser | ad 100 |

[8] 20 % Aktivsubstanz in Wasser

4.7. Haarshampoo

| Komponenten | Gew.-% |
|---|---|
| Texapon$^R$N 25 | 45,0 |
| Dehyton$^R$K | 15,0 |
| Akypo$^{(R)}$RLM 100 NV | 5,0 |
| Polymerisat P1[8] | 5,0 |
| Ajidew$^R$A 100[12] | 2,0 |
| Wasser | ad 100 |

[8] 20 % Aktivsubstanz in Wasser
12 dl-2-Pyrrolidon-5-carbonsäure (AJINOMOTO)

4.8. Haarshampoo

| Komponenten | Gew.-% |
|---|---|
| Texapon$^R$K14 S spez. | 15,0 |
| Texapon$^R$SB 3 | 12,0 |
| ethoxylierte(9 EO)Palmkernölfettsäure | 1,0 |
| Alkylglucosid APG-600 | 4,0 |
| Dehyton$^R$CB[13] | 9,7 |
| Polymerisat P1[8] | 3,0 |
| Ajidew$^R$N50 | 2,0 |
| Wasser | ad 100 |

[8] 20 % Aktivsubstanz in Wasser
[13] Wäßrige Lösung eines Fettamin-Derivates mit Betainstruktur; CTFA-Bezeichnung: Coco-Betaine (ca. 31 % Aktivsubstanz, ca. 6,5 % NaCl) (HENKEL)

4.9. Haarshampoo

| Komponenten | Gew.-% |
|---|---|
| Texapon^R SB 3 | 12,0 |
| ethoxylierte(9 EO)Palmkernölfettsäure | 1,0 |
| Dehyton^R CB | 10,0 |
| Eucarol^(R) TA[14] | 20,0 |
| Polymerisat P1[8] | 1,2 |
| Ajidew^R N50 | 3,0 |
| Wasser | ad 100 |

[8] 20 % Aktivsubstanz in Wasser
[14] Wäßrige Lösung von Natriumlaureth-7-tartrat (25 % Aktivsubstanz) (ROL)

2.10. Haarshampoo

| Komponenten | Gew.-% |
|---|---|
| Texapon^R SB 3 | 15,0 |
| Dehyton^R CB | 12,0 |
| Alkylglucosid APG-600 | 4,0 |
| Polymerisat P1[8] | 1,2 |
| Ajidew^R N50 | 3,0 |
| Wasser | ad 100 |

[8] 20 % Aktivsubstanz in Wasser

**Patentansprüche**

**1.** Zubereitungen, enthaltend übliche Bestandteile zur Behandlung der Haare, dadurch gekennzeichnet, das sie eine Kombination aus

A) einem zwitterionischen Polymerisat und

B) einer Verbindung der allgemeinen Formel (I),

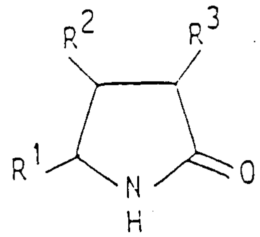

( I )

in der mindestens einer der Substituenten $R^1$ bis $R^3$ steht für eine Gruppe -$COOR^4$, in der $R^4$ Wasserstoff, ein Alkalimetallion, ein Erdalkalimetallion oder ein Ammoniumion $^+NHR^5R^6R^7$, in dem $R^5$ bis $R^7$ unabhängig voneinander Wasserstoff, Alkylgruppen mit 1 bis 22 C-Atomen, Hydroxyalkylgruppen mit 1 bis 4 C-Atomen, Alkenylgruppen mit 2 bis 22 C-Atomen, Acylgruppen mit 2 bis 22 C-Atomen oder gegebenenfalls substituierte aromatische Gruppen mit 6 bis 10 C-Atomen sind, ist, und die restlichen Substituenten $R^1$ bis $R^3$ für Wasserstoff oder Alkylgruppen mit 1 bis 4 C-Atomen stehen, enthalten.

**2.** Zubereitungen nach Anspruch 1, dadurch gekennzeichnet, daß sich die zwitterionischen Polymerisate im wesentlichen zusammensetzen aus

α) Monomeren mit quartären Ammoniumgruppen der allgemeinen Formel (II),

$$R^8\text{-CH=CR}^9\text{-CO-Z-}(C_nH_{2n})\text{-N}^{(+)}R^{10}R^{11}R^{12}\ A^{(-)} \qquad (II)$$

in der $R^8$ und $R^9$ unabhängig voneinander stehen für Wasserstoff oder eine Methylgruppe und $R^{10}$, $R^{11}$ und $R^{12}$ unabhängig voneinander für Alkylgruppen mit 1 bis 4 Kohlenstoff-Atomen, Z eine NH-Gruppe oder ein Sauerstoffatom, n eine ganze Zahl von 2 bis 5 und $A^{(-)}$ das Anion einer organischen oder anorganischen Säure ist

und

β) monomeren Carbonsäuren der allgemeinen Formel (III),

$$R^{13}\text{-CH=CR}^{14}\text{-COOH} \qquad (III)$$

in denen $R^{13}$ und $R^{14}$ unabhängig voneinander Wasserstoff oder Methylgruppen sind, oder Alkali-, Erdalkali-, Aluminium- oder Ammoniumsalzen dieser Säuren.

**3.** Zubereitungen nach einem der Ansprüche 1 oder 2, dadurch gekennzeichnet, daß das Monomere des Typs (α) Acrylamidopropyl-trimethyl-ammoniumchlorid und das Monomere des Typs (β) Acrylsäure oder ein Alkalisalz, insbesondere das Natriumsalz, der Acrylsäure ist.

**4.** Zubereitungen nach einem der Ansprüche 1 bis 3, dadurch gekennzeichnet, daß die Verbindung (B) 2-Pyrrolidon-5-carbonsäure oder ein Salz dieser Säure ist.

**5.** Zubereitungen nach einem der Ansprüche 1 bis 4, dadurch gekennzeichnet, daß die Verbindung (B) das Natriumsalz der 2-Pyrrolidon-5-carbonsäure ist.

**6.** Zubereitungen nach einem der Ansprüche 1 bis 5, dadurch gekennzeichnet, daß sie die zwitterionischen Polymerisate in Mengen von 0,1 bis 10 Gew.-%, insbesondere von 0,2 bis 5 Gew.-%, bezogen auf die gesamte Zubereitung, enthalten.

**7.** Zubereitungen nach einem der Ansprüche 1 bis 6, dadurch gekennzeichnet, daß sie die Verbindung der Formel (I) in Mengen von 0,1 bis 15 Gew.-%, insbesondere von 0,5 bis 10 Gew.-%, bezogen auf die gesamte Zubereitung, enthalten.

**8.** Zubereitungen nach einem der Ansprüche 1 bis 7, dadurch gekennzeichnet, daß sie zusätzlich ein nichtionisches Tensid enthalten.

**9.** Zubereitungen nach Anspruch 8, dadurch gekennzeichnet, daß sie als nichtionisches Tensid ein $C_8$-$C_{22}$-Alkylmono- und -oligoglucosid in einer Menge von 0,5 bis 10 Gew.-%, bezogen auf die gesamte Zubereitung, enthalten.

**10.** Zubereitungen nach einem der Ansprüche 1 bis 9, dadurch gekennzeichnet, daß sie als Shampoo oder Haarspülung formuliert sind.

**11.** Zubereitungen nach einem der Ansprüche 1 bis 10, dadurch gekennzeichnet, daß sie einen pH-Wert von 3,5 bis 10, insbesondere von 4,5 bis 9, aufweisen.

**12.** Verwendung von Zubereitungen nach einem der Ansprüche 1 bis 11 zur Behandlung, insbesondere zur Reinigung und Pflege, von Haaren.

**13.** Verwendung nach Anspruch 12, dadurch gekennzeichnet, daß das Haar nach der Anwendung der Zubereitung gespült wird.

**Claims**

**1.** Preparations containing typical constituents for the treatment of hair, characterized in that they contain a combination of

A) a zwitterionic polymer and
B) a compound corresponding to general formula (I):

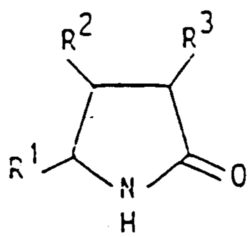

(I)

in which at least one of the substituents $R^1$ to $R^3$ is a group -COOR$^4$, where $R^4$ is hydrogen, an alkali metal ion, an alkaline earth metal ion or an ammonium ion $^+NHR^5R^6R^7$, where $R^5$ to $R^7$ independently of one another represent hydrogen, alkyl groups containing 1 to 22 carbon atoms, hydroxyalkyl groups containing 1 to 4 carbon atoms, alkenyl groups containing 2 to 22 carbon atoms, acyl groups containing 2 to 22 carbon atoms or optionally substituted aromatic groups containing 6 to 10 carbon atoms, and the remaining substituents $R^1$ to $R^3$ represent hydrogen or alkyl groups containing 1 to 4 carbon atoms.

**2.** Preparations as claimed in claim 1, characterized in that the zwitterionic polymers are essentially composed of:

α) monomers containing quaternary ammonium groups corresponding to general formula (II):

$$R^8\text{-CH=CR}^9\text{-CO-Z-}(C_nH_{2n})\text{-N}^{(+)}R^{10}R^{11}R^{12}\ A^{(-)} \qquad (II)$$

in which $R^8$ and $R^9$ independently of one another represent hydrogen or a methyl group and $R^{10}$, $R^{11}$ and $R^{12}$ independently of one another represent alkyl groups containing 1 to 4 carbon atoms, Z is an NH group or an oxygen atom, n is an integer of 2 to 5 and $A^{(-)}$ is the anion of an organic or inorganic acid

and

β) monomeric carboxylic acids corresponding to general formula (III):

$$R^{13}\text{-CH=CR}^{14}\text{-COOH} \qquad (III)$$

in which $R^{13}$ and $R^{14}$ independently of one another represent hydrogen or methyl groups, or alkali metal, alkaline earth metal, aluminium or ammonium salts of these acids.

3. Preparations as claimed in claim 1 or 2, characterized in that the monomer ($\alpha$) is acrylamidopropyl trimethyl ammonium chloride while the monomer ($\beta$) is acrylic acid or an alkali metal salt thereof, more particularly the sodium salt.

4. Preparations as claimed in any of claims 1 to 3, characterized in that compound (B) is 2-pyrrolidone-5-carboxylic acid or a salt thereof.

5. Preparations as claimed in any of claims 1 to 4, characterized in that compound (B) is the sodium salt of 2-pyrrolidone-5-carboxylic acid.

6. Preparations as claimed in any of claims 1 to 5, characterized in that they contain the zwitterionic polymers in quantities of 0.1 to 10% by weight and, more particularly, in quantities of 0.2 to 5% by weight, based on the preparation as a whole.

7. Preparations as claimed in any of claims 1 to 6, characterized in that they contain the compound corresponding to formula (I) in quantities of 0.1 to 15% by weight and more particularly in quantities of 0.5 to 10% by weight, based on the preparation as a whole.

8. Preparations as claimed in any of claims 1 to 7, characterized in that they additionally contain a non-ionic surfactant.

9. Preparations as claimed in claim 8, characterized in that they contain a $C_{8-22}$ alkyl mono- and -oligoglucoside in a quantity of 0.5 to 10% by weight, based on the preparation as a whole, as the nonionic surfactant.

10. Preparations as claimed in any of claims 1 to 9, characterized in that they are formulated as a shampoo or hair rinse.

11. Preparations as claimed in any of claims 1 to 10, characterized in that they have a pH value of 3.5 to 10 and, more particularly, 4.5 to 9.

12. The use of the preparations claimed in any of claims 1 to 11 for the treatment of hair, more particularly for the washing and care of hair.

13. The use claimed in claim 12, characterized in that the hair is rinsed after application of the preparation.

**Revendications**

1. Préparations contenant des constituants usuels pour le traitement des cheveux,
caractérisées en ce qu'
elles renferment une combinaison à base de :

   A) un polymérisat zwitterionique et,
   B) un composé de formule générale (I)

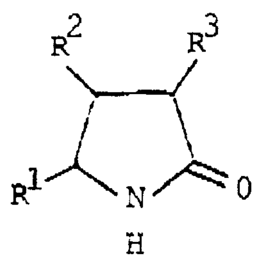

(I)

dans laquelle au moins un des substituants $R^1$ à $R^3$ représente un groupe-$COOR^4$, dans lequel $R^4$ est un hydrogène, un ion de métal alcalin, un ion de métal alcalino-terreux, ou un ion ammonium $^+NHR^5R^6R^7$, dans lequel $R^5$ à $R^7$ indépendamment l'un de l'autre sont de l'hydrogène, des radicaux alcoyle ayant de 1 à 22 atomes de carbone, des radicaux hydroxyalcoyle ayant de 1 à 4 atomes de carbone, des radicaux alcényle ayant de 2 à 22 atomes de carbone, des groupes acyle ayant de 2 à 22 atomes de carbone, ou des groupes aromatiques éventuellement substitués ayant de 6 à 10 atomes de carbone et les substituants restant $R^1$ à $R^3$ représentent de l'hydrogène, ou des radicaux alcoyle ayant de 1 à 4 atomes de carbone.

**2.** Préparations selon la revendication 1,
caractérisées en ce que
les polymérisats zwitterioniques sont formés essentiellement de :

α) de monomères avec des groupes d'ammonium quaternaires de formule générale (II)

$$R^8\text{-}CH{=}CR^9\text{-}CO\text{-}Z\text{-}(C_nH_{2n})\text{-}N^{(+)}R^{10}R^{11}R^{12}\ A^{(-)} \quad (II)$$

dans laquelle $R^8$ et $R^9$ indépendamment l'un de l'autre, représentent de l'hydrogène ou un radical méthyle et $R^{10}$, $R^{11}$ et $R^{12}$ indépendamment l'un de l'autre, représentent des radicaux alcoyle ayant de 1 à 4 atomes de carbone, Z est un groupe -NH ou un atome d'oxygène, n un nombre entier allant de 2 à 5 et $(A^-)$ l'anion d'un acide organique ou minéral et,
β) des monomères d'acides carboxyliques monomères de formule générale (III),

$$R^{13}\text{-}CH{=}CR^{14}\text{-}COOH \quad (III)$$

dans laquelle $R^{13}$ et $R^{14}$ indépendamment l'un de l'autre sont de l'hydrogène ou des groupes méthyle

ou bien des sels de métal alcalin, alcalino-terreux, d'aluminium ou d'ammonium de ces acides.

**3.** Préparations selon l'une des revendications 1 ou 2,
caractérisées en ce que
le monomère du type α est le chlorure d'acrylamidopropyltriméthyl-ammonium et le monomère du type β est l'acide acrylique ou un sel de métal alcalin, en particulier le sel de sodium, d'acide acrylique.

**4.** Préparations selon l'une des revendications 1 à 3,
caractérisées en ce que
le composé (B) est l'acide 2-pyrrolidone-5-carboxylique ou un sel de cet acide.

**5.** Préparations selon l'une des revendications 1 à 4,
caractérisées en ce que
le composé (B) est le sel de sodium de l'acide 2-pyrrolidone-5-carboxylique.

**6.** Préparations selon l'une des revendications 1 à 5,
caractérisées en ce qu'
elles renferment les polymérisats zwitterioniques en quantités allant de 0,1 à 10 % en poids, en particulier de 0,2 à 5 % en poids, rapporté à la préparation globale.

**7.** Préparations selon l'une des revendications 1 à 6,
caractérisées en ce qu'
elles renferment le composé de formule (I) en quantités allant de 0,1 à 15 % en poids, en particulier de 0,5 à 10 % en poids, rapporté à la préparation globale.

**8.** Préparations selon l'une des revendications 1 à 7,
caractérisées en ce qu'
elles renferment en supplément un agent tensioactif non ionique.

**9.** Préparations selon la revendication 8,
caractérisées en ce qu'
elles renferment comme agent tensioactif non ionique un (alcoyle en $C_8$ à $C_{22}$) mono-et oligoglucoside en quantité allant de 0,5 à 10 % en poids, rapporté à la préparation globale.

**10.** Préparations selon l'une des revendications 1 à 9, caractérisées en ce qu' elles sont formulées sous la forme de shampooing ou de rinçage pour les cheveux.

**11.** Préparations selon l'une des revendications 1 à 10, caractérisées en ce qu' elles possèdent une valeur pH de 3,5 à 10, en particulier de 4,5 à 9.

**12.** Utilisation de préparations selon l'une des revendications 1 à 11 pour le traitement, en particulier pour le nettoyage et les soins des cheveux.

**13.** Utilisation selon la revendication 12, caractérisée en ce que les cheveux sont rincés après l'application de la préparation.